# EUROPÄISCHE PATENTSCHRIFT

(11) **EP 0 629 187 B1**
(45) Veröffentlichungstag und Bekanntmachung des Hinweises auf die Patenterteilung: **23.04.1997**
(21) Anmeldenummer: 93905268.4
(22) Anmeldetag: 22.02.1993
(51) Int. Cl.: C07C 309/14, C07C 303/22, C09B 51/00, A61K 7/13

(54) **DIREKTZIEHENDE FARBSTOFFE FÜR FASERN NATÜRLICHEN URSPRUNGS UND SYNTHETISCHE FASERN**
DIRECT-DYEING COLOURING AGENTS FOR NATURAL AND SYNTHETIC FIBRES
MATIERES COLORANTES MONTANT DIRECTEMENT SUR LA FIBRE, POUR FIBRES D'ORIGINE NATURELLE ET POUR FIBRES SYNTHETIQUES

(30) Priorität: 02.03.1992 DE 4206536
(43) Veröffentlichungstag der Anmeldung: 21.12.1994
(73) Patentinhaber: Henkel Kommanditgesellschaft auf Aktien, 40191 Düsseldorf (DE)
(72) Erfinder: ROSE, David, D-4010 Hilden (DE); HÖFFKES, Horst, D-4000 Düsseldorf (DE); LIESKE, Edgar, D-4000 Düsseldorf (DE)
(86) Internationale Anmeldenummer: EP9300416
(87) Internationale Veröffentlichungsnummer: WO9317999

(56) Entgegenhaltungen:
- DD-A- 222 013
- DE-A- 3 616 720
- DE-B- 1 114 775
- FR-A- 1 575 821

## Beschreibung

Gegenstand der Erfindung ist ein Verfahren zur Herstellung von N-(2-Nitro-4-aminophenyl)-aminoalkansulfonsäuren sowie deren Verwendung zum Färben von Fasern natürlichen Ursprungs und synthetischen Fasern.

Die direktziehenden Farbstoffe spielen in der Färberei eine herausragende Rolle. Direktziehende Farbstoffe sind wasserlösliche Verbindungen, die aus einer wäßrigen Lösung oder einer wasserhaltigen Zubereitung auf die Faser aufziehen. Sie müssen über zwei wichtige Strukturmerkmale verfügen: ein chromophores System und eine oder mehrere hydrophile Gruppen, die ihnen eine ausreichende Wasserlöslichkeit verleihen.

In der Klasse der Nitrofarbstoffe sind diejenigen von Bedeutung, die an einem aromatischen System neben einer oder mehreren Nitrogruppen Hydroxy- oder Aminogruppen tragen; diese Kombination aus Nitrosubstituenten und elektronenreichen Gruppen ist für die Farbigkeit der Verbindungen verantwortlich.

Eine ausreichende Wasserlöslichkeit wird durch Substitution mit polaren Gruppen erreicht. Bevorzugt wird hier der Sulfonsäure- bzw.

Sulfonatrest verwendet, dabei ist der Sulfonsäurerest im allgemeinen direkt an den Aromaten gebunden. Die resultierenden Benzolsulfonsäurederivate unterscheiden sich von den Phenylalkansulfonsäuren und Phenylaminoalkansulfonsäuren dadurch, daß bei letzteren die solubilisierende Gruppe über Alkyl- bzw. Aminoalkylreste an den Aromaten gebunden ist.

Zum Färben von Haaren und Pelzen sind in der deutschen Auslegeschrift DE 11 14 775 bereits N-(Nitro-4-aminophenyl)-aminoalkansulfonsäuren und deren Salze beschrieben. Die damit erzielten Färbungen sind jedoch nur wenig farbintensiv. Außerdem hat das Verfahren zur Herstellung der dort beschriebenen Verbindungen gravierende Nachteile: Nach diesem Verfahren wird Nitroparaphenylendiamin in Benzol mit Propansulton zu N-(Nitro-4-aminophenyl)-3-amino-1-propansulfonsäure umgesetzt. Auf die Verwendung der karzinogenen Chemikalien Benzol und Propansulton sollte nach heutigen Erkenntissen jedoch möglichst verzichtet werden. Ein weiterer Nachteil dieses Syntheseprinzips liegt darin, daß der Verlängerung der Alkansulfonsäurekette Grenzen gesetzt sind, da die höherhomologen Alkansultone nicht mehr gut zugänglich sind. Weiterhin wird nach diesem Verfahren ein Isomerengemisch erhalten, das überwiegend N-(3-Nitro-4-aminophenyl)-3-amino-1-propansulfonsäure enthält. Das 3-Nitro-Isomere führt jedoch nur zu unbefriedigenden Färbeergebnissen. Außerdem werden für die heutigen strengeren toxikologischen Prüfungen isomerenreine Produkte gefordert.

Aufgabe der Erfindung ist es daher, ein Verfahren zur Herstellung der als Färbemittel geeigneten N-(2-Nitro-4-aminophenyl)-aminoalkansulfonsäuren ohne die oben beschriebenen Nachteile zu finden.

Es wurde nun ein Verfahren zur Herstellung von N-(2-Nitro-4-aminophenyl)-aminoalkansulfonsäuren der Formel I gefunden 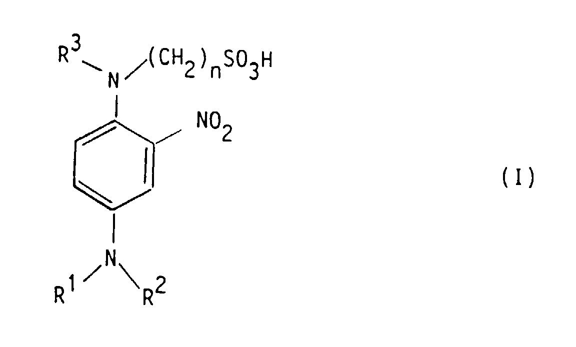 wobei R¹, R² und R³ Wasserstoff, Alkylgruppen mit 1 bis 4 C-Atomen oder Hydroxyalkylgruppen mit 2 bis 4 C-Atomen darstellen und n eine ganze Zahl von 1 bis 6 ist und deren Salzen, indem man Verbindungen der Formel II 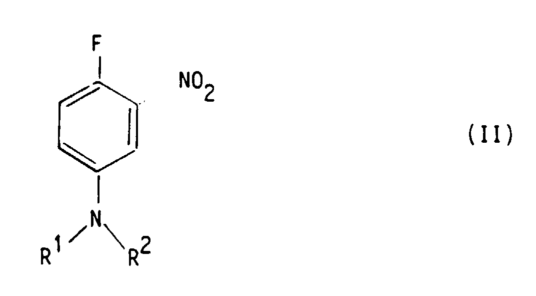 mit Verbindungen der Formel III,

R³N(H) - (CH₂)ₙ - SO₃H (III),

umsetzt, wobei R¹ und R² in Formel II bzw. R³ und n in Formel III die gleiche Bedeutung wie in Formel I haben.
Bevorzugt anzuwenden ist das Verfahren, wenn in Formel I, II und III R¹ und R² Wasserstoff, R³ Wasserstoff oder Methyl bedeuten und n gleich 2 oder 3 ist.
Unter den wasserlöslichen Salzen sind in erster Linie die Salze starker Basen, wie etwa die Alkalisalze, z. B. das Natrium- oder Kaliumsalz, oder die Ammoniumsalze zu verstehen.

Vorzugsweise wird dem Reaktionsgemisch zur Neutralisierung der bei der Reaktion freiwerdenden Fluorwasserstoffsäure eine basische Verbindung, z. B. Soda oder Pottasche, zugesetzt.

Nach dem erfindungsgemäßen Verfahren kann auf die Verwendung von hochtoxischen oder karzinogenen Verbindungen verzichtet werden. Die Verbindungen der Formel I werden durch einfaches Fällen oder Auskristallisieren aus der Reaktionsmischung in hoher Reinheit enthalten. Es werden ausschließlich die 2-Nitroisomeren gebildet. Der Forderung nach isomerenreinen Produkten wird damit Rechnung getragen. Außerdem steht nach dem erfindungsgemäßen Verfahren der Weg zu Verbindungen der Formel I mit Alkansulfonsäureketten mit mehr als 4 C-Atomen offen.

Als Lösungsmittel können alle üblichen organischen Lösungsmittel eingesetzt werden, vorzugsweise werden jedoch hochsiedende Lösungsmittel, wie z. B. Dimethylsulfoxid, verwendet oder aber Wasser.

Die N-(2-Nitro-4-aminophenyl)-aminoalkansulfonsäuren der Formel I und deren Salze eignen sich hervorragend zum Färben von Fasern natürlichen Ursprungs und synthetischen Fasern.

Erfindungsgemäß sind unter Fasern natürlichen Ursprungs menschliche, tierische und pflanzliche Fasern, wie Seide, Baumwolle, Leinen, Jute und Sisal und Keratinfasern, wie Haare, Pelze, Wolle oder Federn aber auch regenerierte oder modifizierte Naturfasern, wie z. B. Viskose, Nitro- und Acetylcellulose, Alkyl-, Hydroxyalkyl- und Carboxyalkylcellulosen zu verstehen. Aus der Klasse der synthetischen Fasern sind z. B. Polyamid-, Polyester-, Polyacrylnitril- und Polyurethanfaser zu nennen.

Ein weiterer Erfindungsgegenstand ist deshalb die Verwendung von N-(2-Nitro-4-aminophenyl)-aminoalkansulfonsäuren der Formel I und deren Salzen zum Färben von Fasern natürlichen Ursprungs und synthetischen Fasern. Die Verbindungen der Formel I werden als Direktfarbstoffe eingesetzt. Textilfasern werden dabei bevorzugt nach dem Ausziehverfahren aus einer wäßrigen, zur besseren Löslichkeit der Verbindungen der Formel I vorzugsweise alkalisch eingestellten Färbeflotte bei Temperaturen über 90 °C gefärbt.

Die besten Färbeergebnisse werden mit keratinischen Fasern erzielt. Wolle beispeilsweise wird in intensiven Farbtönen von kamelbraun bis rotbraun eingefärbt.

Eine weitere in der Färberei bedeutsame keratinhaltige Faser ist das menschliche Haar. Auch menschliches Haar kann mit den Verbindungen der Formel I bereits bei physiologisch zuträglichen Temperaturen unterhalb 40 °C intensiv eingefärbt werden.

Ein weiterer Erfindungsgegenstand ist deshalb die Verwendung von N-(2-Nitro-4-aminophenyl)-aminoalkansulfonsäuren der Formel I und deren Salzen zum Färben von keratinhaltigen Fasern, vorzugsweise menschlichem Haar.

Zum Färben von menschlichen Haaren werden die Verbindungen der Formel I in einem wäßrigen kosmetischen Träger, z. B. in Cremes, Emulsionen, Gele oder tensidhaltige, schäumende Lösungen, z. B. in Shampoos, Schaumaerosole oder anderen Zubereitungen, die für die Anwendung auf dem Haar geeignet sind, eingearbeitet.

Übliche Bestandteile solcher kosmetischer Zubereitungen sind z. B. Netz- und Emulgiermittel, wie anionische, nichtionische oder ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel, wie z. B. Methyl- oder Hydroxyethylcellulose, Stärke, Fettalkohole, Paraffinöle, Fettsäuren, ferner Parfumöle und haarpflegende Zusätze, wie z. B. wasserlösliche kationische Polymere, Proteinderivate, Pantothensäure und Cholesterin. Die Bestandteile der kosmetischen Träger werden zur Herstellung von Haarfärbemitteln in für diese Zwecke üblichen Mengen eingesetzt. Z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Zur Anwendung in Haarfärbemitteln werden die direktziehenden Farbstoffe der Formel I in einer Menge von 0,01 bis 5 Gew.-%, bevorzugt von 0,1 bis 2 Gew.-%, bezogen auf die gesamte Zubereitung, eingesetzt.

Die Färbetemperaturen liegen in diesem Fall erheblich niedriger als in der Textilfärberei. Sie sollten 40 °C nicht übersteigen. Die mit den Verbindungen der Formel I erzielten Haarfärbungen liegen im Bereich von kamelbraun bis rosarot und unterscheiden sich damit deutlich von dem in der deutschen Auslegeschrift DE 11 14 775 beschriebenen hellbraunen Farbton. Sie verfügen außerdem über hohe Farbintensitäten, Licht- und Waschechtheiten.

Zur Farbmodifikation können die Verbindungen der Formel I mit üblichen bekannten direktziehenden Farbstoffen kombiniert eingesetzt werden. Daneben können auch bekannte Oxidationshaarfärbemittelvorprodukte enthalten sein. Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2 Gew.-% Natriumsulfit zur Stabilisierung der Oxidationsfarbstoffvorprodukte. In diesem Fall wird vor der Anwendung des Haarfärbemittels dieses mit einem Oxidationsmittel versetzt um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der Haarfärbemittel kann unabhängig von der Art der kosmetischen Zubereitung, z. B. als Creme, Gel oder Shampoo, im schwach sauren, neutralen oder alkalischen Milieu erfolgen. Bevorzugt ist die Anwendung der Haarfärbemittel in einem pH-Bereich von 6 bis 10.
Die nachfolgenden Beispiele sollen den Erfindungsgegenstand näher erläutern, ohne ihn jedoch hierauf zu beschränken.

### Beispiele

### Herstellungsbeispiele

1. N-(2-Nitro-4-aminophenyl)-2-amino-1-ethansulfonsäure (F1)
   Eine Mischung bestehend aus 12,5 g (0,1 Mol) 2-Amino-1-ethansulfonsäure (Taurin) und 6,9 g (0,05 Mol) Kaliumcarbonat in 20 ml Dimethylsulfoxid wurde auf 110 °C erhitzt. Eine Lösung von 7,8 g (0,05 Mol) 4-Fluor-3-nitroanilin in 20 ml Dimethylsulfoxid wurde bei dieser Temperatur innerhalb von 90 Minuten zugetropft. Nach dem Abkühlen wurde das Reaktionsgemisch in 400 ml Wasser gegossen. Die Lösung wurde mit verdünnter Salzsäure auf pH 3 eingestellt. Nach dem Abkühlen wurde das Produkt absaugt, mit Wasser nachgewaschen und im Vakuum bei 60 °C getrocknet.
   Ausbeute: 6,35 g (48,7 % der Theorie)
   Es wurde ein UV/VIS Spektrum in Ethanol als Lösungsmittel, mit einem Gehalt von 1 % 10 %igem Ammoniak, aufgenommen: ₘₐₓ = 247 nm, 294 nm.
2. N-(2-Nitro-4-aminophenyl)-3-amino-1-propansulfonsäure (F2)
   Eine Mischung bestehend aus 3,9 g (0,025 Mol) 4-Fluor-3-nitroanilin und 3,8 g (0,025 Mol) 3-Amino-1-propansulfonsäure und 10,4 g (0,075 Mol) Kaliumcarbonat in 40 ml Wasser wurde 5 Stunden lang unter Rückfluß gekocht. Nach dem Abkühlen wurde mit konzentrierter Salzsäure auf pH 2 eingestellt. Das Produkt wurde abgesaugt, mit Wasser nachgewaschen und im Vakuum bei 60 °C getrocknet.
   Man erhielt schwarze Kristalle.
   Ausbeute: 1,85 g (26,8 % der Theorie)
   Es wurde ein UV/VIS-Spektrum in Ethanol als Lösungsmittel, mit einem Gehalt von 1 % 10 %igem Ammoniak, aufgenommen: ₘₐₓ = 247 nm, 486 nm.
3. N-(2-Nitro-4-aminophenyl)-2-methylamino-1-ethansulfonsäure (F3)
   Die Synthese wurde analog zu Beispiel 2 durchgeführt, aber mit 2-Methylamino-1-ethansulfonsäure (N-Methyltaurin) anstatt 3-Amino-1-propansulfonsäure. Man erhielt goldglänzende Kristalle.
   Ausbeute: 5,6 g (81,3 % der Theorie)

### Anwendungsbeispiele

### Färben von Textilfasern

Die Färbungen wurden an einem Mehrfaserbegleitgewebe, Typ DW (Deutsche Echtheitskommission) der Beuth-Verlags-GmbH, Köln, bestehend aus 6 Textilstreifen aus Acetylcellulosefasern (2,5 Acetylgruppen pro Monomereinheit), Baumwollfasern, Polyamidfasern, Polyesterfasern, Polyacrylfasern und Wollfasern durchgeführt.

0,5 g des Farbstoffs, 8 g Natriumsulfatdeckerhydrat und 2 g Natriumcarbonat wurden bei 60 °C in 100 ml Wasser gelöst. Dem Färbebad wurde das Mehrfaserbegleitgewebe zugegeben. Anschließend wurde innerhalb von 45 Minuten auf 48 °C erhitzt. Diese Temperatur wurde 1 Stunde lang beibehalten, wobei verdampftes Wasser kontinuierlich ersetzt wurde. Das gefärbte Material wurde anschließend dem Färbebad entnommen und zunächst kalt und dann heiß gespült. Das gefärbte Mehrfasergewebe wurde dann 20 Minuten lang in einer Mischung aus 250 ml Wasser und 0,25 g Natriumlaurylsulfat gekocht. Anschließend wurde das Gewebe gespült und getrocknet. Die mit den Farbstoffen 1 bis 3 erzielten Färbungen sind Tabelle 1 zu entnehmen.

**Tabelle 1**

| Fasertyp | Farbstoff 1 | Farbstoff 2 | Farbstoff 3 |
|---|---|---|---|
| Acetylcellulose | dunkelbraun | hellbraun | braunorange |
| | | | |
| Baumwolle | rehbraun | rehbraun | rotgrau |
| | | | |
| Polyamid | braunorange | rotbraun | rotweiß |
| | | | |
| Polyester | nicht gefärbt | graurot | orangeweiß |
| | | | |
| Polyacryl | nicht gefärbt | mattrot | orangeweiß |
| | | | |
| Wolle | hellbraun | rotbraun | kamelbraun |

### Färben von menschlichen Haaren

Es wurden Haarfärbecremes der folgenden Zusammensetzung hergestellt:

| | |
|---|---|
| Fettalkohol C₁₂₋₁₈ | 10 g |
| Fettalkohol C₁₂₋₁₄ + 2 EO Sulfat, Na-Salz (28 %ig) | 25 g |
| Wasser | 60 g |
| direktziehender Farbstoff (F1 bis F3) | 7,5 mMol |
| Ammoniumsulfat | 1 g |
| konz. Ammoniaklösung | bis pH 9,5 |
| Wasser | ad 100 g |

Die Bestandteile wurden der Reihe nach miteinander vermischt. Nach Zugabe der direktziehenden Farbstoffe wurde mit konz. Ammoniaklösung der pH-Wert der Emulsion auf 9,5 eingestellt. Dann wurde mit Wasser auf 100 g aufgefüllt. Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet. Die Ergebnisse der Färbeversuche sind Tabelle 2 zu entnehmen.

**Tabelle 2**

| Farbstoff | Nuance des gefärbten Haares |
|---|---|
| F1 | rosarot |
| | |
| F2 | mattrot |
| | |
| F3 | kamelbraun |

## Patentansprüche

1. Verfahren zur Herstellung von N-(2-Nitro-4-aminophenyl)-aminoalkansulfonsäuren der Formel I 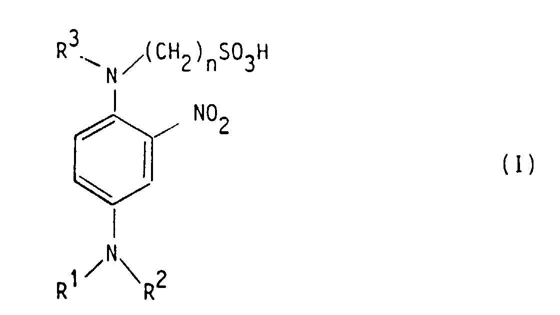 wobei R¹, R² und R³ Wasserstoff, Alkylgruppen mit 1 bis 4 C-Atomen oder Hydroxyalkylgruppen mit 2 bis 4 C-Atomen darstellen und n eine ganze Zahl von 1 bis 6 ist und deren Salzen durch Umsetzung von Verbindungen der Formel II 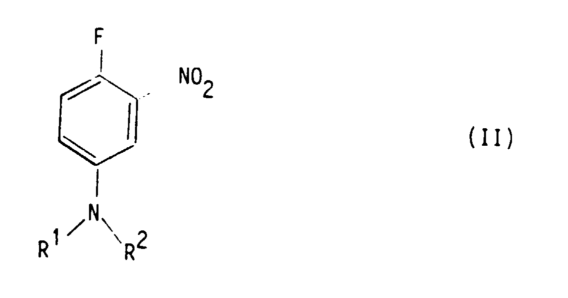 mit Verbindungen der Formel III
R³N(H)-(CH₂)ₙ - SO₃H (III),
wobei R¹ und R² in Formel II bzw. R³ und n in Formel III die gleiche Bedeutung wie in Formel I haben.

2. Verfahren nach Ansprch 1, wobei in Formel I, II und III R¹ und R² Wasserstoff, R³ Wasserstoff oder Methyl bedeuten und n = 2 oder 3 ist.

3. Verwendung von N-(2-Nitro-4-aminophenyl)-aminoalkansulfonsäuren der Formel I und deren Salzen zum Färben von Fasern natürlichen Ursprungs und synthetischen Fasern.

4. Verwendung von N-(2-Nitro-4-aminophenyl)-aminoalkansulfonsäuren der Formel I und deren Salzen nach Anspruch 3 zum Färben von keratinhaltigen Fasern, vorzugsweise menschlichem Haar.

## Claims

1. A process for the production of N-(2-nitro-4-aminophenyl)-aminoalkane sulfonic acids corresponding to formula I: 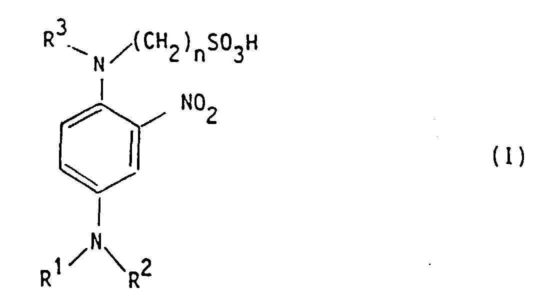 in which R¹, R² and R³ are hydrogen, alkyl groups containing 1 to 4 carbon atoms or hydroxyalkyl groups containing 2 to 4 carbon atoms and n is an integer of 1 to 6,
and salts thereof by reacting compounds corresponding to formula II: 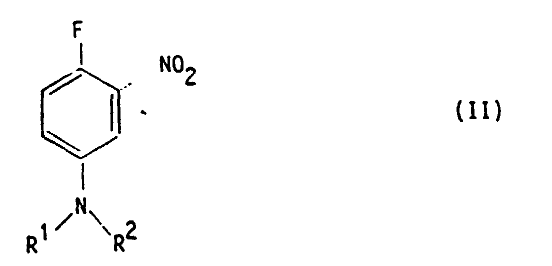 with compounds corresponding to formula III:
R³N(H)-(CH₂)ₙ - SO₃H (III),
R¹ and R² in formula II and R³ and n in formula III having the same meanings as in formula I.

2. A process as claimed in claim 1, R¹ and R² in formulae I, II and III being hydrogen, R³ being hydrogen or methyl and n = 2 or 3.

3. The use of N-(2-nitro-4-aminophenyl)-aminoalkane sulfonic acids corresponding to formula I and salts thereof for colouring fibres of natural origin and synthetic fibres.

4. The use of N-(2-nitro-4-aminophenyl)-aminoalkane sulfonic acids corresponding to formula I and salts thereof according to claim 3 for colouring keratin-containing fibres, preferably human hair.

## Revendications

1. Procédé de préparation des acides N-(2-nitro-4-aminophényl)-aminoalcanesulfoniques de formule (I). 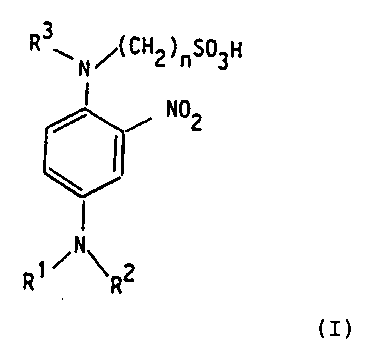 où R¹, R² et R³ représentent un hydrogène, des groupes alkyle de 1 à 4 atomes de C ou des groupes hydroxyalkyle de 2 à 4 atomes de C et n est un nombre entier de 1 à 6 et de leurs sels par réaction de composés de formule II : 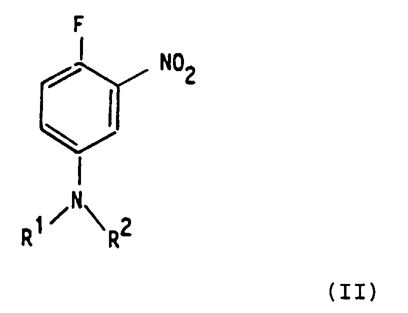 Avec des composés de formule III
R³N(H) - (CH₂)ₙ - SO₃H (III),
où R¹ et R² dans la formule II, respectivement R³ et n, dans la formule III, ont les mêmes significations qu'à la formule I.

2. Procédé selon la revendication 1, où dans les formules I, II et III R¹ et R² représentent de l'hydrogène, R³ représente de l'hydrogène ou du méthyle et n = 2 ou 3.

3. Utilisation d'acides N-(2-nitro-4-aminophényl)-aminoalcanesulfoniques de formule I et de leurs sels pour teindre des fibres d'origine naturelle et des fibres synthétiques.

4. Utilisation d'acides N-(2-nitro-4-aminophényl)-aminoalcanesulfoniques de formule I et de leurs sels selon la revendication 3 pour teindre des fibres contenant de la kératine, de préférence des cheveux humains.
